# EUROPÄISCHE PATENTSCHRIFT

(11) **EP 1 531 784 B1**
(45) Veröffentlichungstag und Bekanntmachung des Hinweises auf die Patenterteilung: **08.09.2010**
(21) Anmeldenummer: 03725189.9
(22) Anmeldetag: 13.05.2003
(51) Int. Cl.: A61Q 5/10, A61K 8/34, A61K 8/42, A61K 8/45, A61K 8/41

(54) **FÄRBEMITTEL MIT PERLGLANZ FÜR KERATINFASERN**
PEARLY-LUSTRE COLORING AGENTS FOR KERATIN FIBERS
AGENTS COLORANTS A LUSTRE NACRE POUR FIBRES KERATINEUSES

(30) Priorität: 31.08.2002 DE 10240276
(43) Veröffentlichungstag der Anmeldung: 25.05.2005
(73) Patentinhaber: The Procter & Gamble Company, Cincinnati, OH 45202 (US)
(72) Erfinder: SCHMENGER, Jürgen, 64331 Weiterstadt (DE); LAUSCHER, Dirk, 64297 Darmstadt (DE); DÖHLING, Annelie, 64839 Münster (DE); KREHER, Helga, 64839 Münster (DE)
(74) Vertreter: Hirsch, Uwe Thomas M.H.
(86) Internationale Anmeldenummer: PCT/EP2003/004961
(87) Internationale Veröffentlichungsnummer: WO 2004/019895

(56) Entgegenhaltungen:
- EP-A- 0 312 343
- EP-A- 0 642 783
- DE-A- 3 834 142
- DE-A- 4 219 981
- DE-C- 19 701 422
- FR-A- 2 817 468
- US-A- 5 376 146

## Beschreibung

Gegenstand der Erfindung sind perlmuttartig glänzende Mittel zum Färben von Keratinfasern, insbesondere menschlichen Haaren, mit einem Gehalt an direktziehenden und/oder oxidativen Farbstoffen und einer speziellen Rohstoffkombination aus langkettigen Fettalkoholen, Alkanolamiden, Fettalkoholalkoxylaten und quaternären Ammoniumverbindungen, ein Verfahren zum Färben von Keratinfasern unter Verwendung dieser Mittel sowie die Verwendung der vorgenannten speziellen Rohstoffkombination zur Erzeugung eines dauerhaften perlmuttartigen Glanzes in Färbemitteln.

Färbende Präparate liegen üblicherweise in Form von wässrigen -vorzugsweise verdickten- Lösungen oder Emulsionen vor und enthalten neben Farbstoffen beispielsweise Fettalkohole und/oder andere Ölkomponenten, Emulgatoren und Tenside, sowie gegebenenfalls Alkohole. Oxidationsfärbemittel bestehen in der Regel aus zwei Komponenten, (i) der die Farbstoffe enthaltenden Farbstoffträgermasse und (ii) der Oxidationsmittelzubereitung, die kurz vor dem Gebrauch miteinandervermischt und dann auf die zu färbende Faser aufgetragen werden. Derartige Farbstoffträgermassen weisen keinen Perlmuttglanz auf, es sei denn es werden entsprechende Perlglanz erzeugende Zusatzstoffe zugesetzt. Zudem verschwindet ein derartiger Perlglanzeffekt beim Vermischen von Farbstoffträgermasse und Oxidationsmittel wieder.

Aus der DE 38 34 142 A1 sind cremeförmige Haarfärbemittel bekannt, welche auf einer komplizierten Mischung einer Vielzahl von Rohstoffen basieren, wobei u.a. auch C14-C20-Fettalkohole, Alkanolamide und Fettalkoholalkoxylate enthalten sind. Diese Mittel enthalten jedoch keine quaternären Ammoniumsalze und weisen auch keinen Perlglanz auf.

Es bestand daher die Aufgabe, eine Farbträgermasse zu entwickeln die alleine durch das Zusammenspiel weniger Rohstoffe und ohne Zusatz von Perlglanz erzeugenden Zusatzstoffen einen beständigen perlmuttartigen Charakter aufweist ("Perlglanzeffekt"), welcher selbst nach dem Vermischen von Oxidationsmittelzubereitung und Farbstoffträgermasse unverändert bestehen bleibt. Eine weitere Aufgabe besteht darin, dass der Pflegeeffekt nach dem Ausspülen der Farbmasse gegenüber üblichen, aus dem Stand der Technik bekannten, Formulierungen erkennbar besser ist.

Überraschenderweise wurde nunmehr gefunden, dass die gestellten Aufgaben durch eine Kombination aus mindestens einem langkettigen Fettalkohol, einem Alkanolamid, einem Fettalkoholalkoxylat und einem quaternären Ammoniumsalz besonders gut gelöst werden.

Gegenstand der vorliegenden Erfindung ist daher ein Färbemittel für Keratinfasern -insbesondere Haare- auf der Basis von Oxidationsfarbstoffvorstufen und/oder direktziehenden Farbstoffen, welches eine Kombination aus
a) mindestens einem langkettigem Fettalkohol, vorzugsweise in einer Menge von 1 bis 20 Gewichtsprozent, insbesondere 2 bis 12 Gewichtsprozent,
b) mindestens einem Alkanolamid, vorzugsweise in einer Menge von 1 bis 20 Gewichtsprozent, insbesondere 2 bis 12 Gewichtsprozent,
c) mindestens einem Fettalkoholalkoxylat, vorzugsweise in einer Menge von 0,1 bis 15 Gewichtsprozent, insbesondere 0,5 bis 10 Gewichtsprozent, und
d) mindestens einer quaternären Ammoniumverbindung der Formel (II) oder (III), 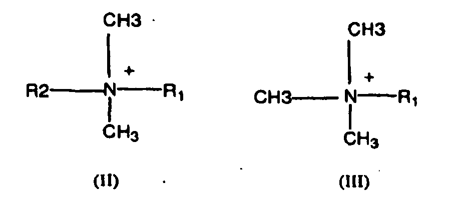
in denen R1 und R2 unabhängig voneinander gleich einer geradkettigen oder verzweigten C8-C24-Alkylgruppe sind (nachfolgend "Ammoniumquat" genannt), vorzugsweise in einer Menge von 0,1 bis 15 Gewichtsprozent, insbesondere 0,5 bis 10 Gewichtsprozent, enthält,
wobei das Mengenverhältnis von Fettalkohol (a) zu Alkanolamid (b) etwa 0,5:1,5 bis 1,5:0,5 beträgt.

Besonders bevorzugt ist hierbei ein Mengenverhältnis von (a) zu (b) von 0,8:1,2 bis 1,2:0,8, bei dem ein besonders schöner Perlmuttcharakter erhalten wird.

Sowohl für die Bildung des Perlmuttcharakters als auch für die Erzielung eines besonders hohen Pflegeeffektes nach dem Ausspülen der Farbmasse ist ein Mengenverhältnis von Fettalkoholalkoxylat (c) zu Ammoniumquat (d) von 0,5:1,5 bis 1,5:0,5, insbesondere 0,8:1,2 bis 1,2:0,8, besonders empfehlenswert.

Als erfindungsgemäß geeignete langkettige Fettalkohole können beispielsweise geradkettige oder verzweigte Fettalkohole mit 8 bis 30 Kohlenstoffatomen, und insbesondere C14- bis C22-Fettalkohole, wie zum Beispiel Isooctylalkohol, Tetradecanol, Cetylakohol, Stearylalkohol, Isotridecylalkohol und Behenylalkohol sowie Mischungen dieser Fettalkohole, genannt werden. Geeignete Fettalkohole werden unter anderem auch von der Firma Cognis, Deutschland unter dem Handelsnamen Lanette® angeboten.

Erfindungsgemäß geeignete Alkanolamide sind beispielsweise Monoalkanolamide, Dialkanolamide oder Esteramide, vorzugsweise N-Acylderivate des Monoethanolamins oder Diethanolamins, wie zum Beispiel Laurinsäuremonoethanolamid, Kokosnussfettsäuremonoethanolamid , Kokosnussfettsäurediethanolamid, Stearinsäuremonoethanolamid, Palmitinsäuremonoethanolamid oder Ölsäuremonoethanolamid sowie deren Mischungen.

Als Fettalkoholethoxylate werden vorzugsweise ethoxylierte Fettalkohole oder Fettalkoholpolyglykoether der nachstehenden Formel (I) eingesetzt.

R-(OCH₂CH₂)ₓ-OH (I)

(mit R = einer geradkettigen oder verzweigten C8-C24-Alkylgruppe oder einer geradkettigen oder verzweigten C8-C24-Alkenylgruppe und x = 2 - 300)

Besonders bevorzugt sind Fettalkoholalkoxylate (Fettalkoholpolyethylenglykolether) der Formel (I) mit R gleich einer geradkettigen oder verzweigten C14-C22-Alkylgruppe und x gleich 2-200 eingesetzt, wie zum Beispiel Polyethylenglykolether des Stearylalkohols mit 10 bis 50 Ethylenoxideinheiten im Molekül (zum Beispiel Steareth-10 oder Steareth-20).

Vorzugsweise werden Ammoniumquats der Formel (II) oder (III) mit R1 und R2 unabhängig voneinander gleich einer geradkettigen oder verzweigten C14-C22-Alkylgruppe oder deren Gemische eingesetzt, wobei Distearyldimethylammoniumchlorid, 2-Hexyldecyltrimethylammoniumchlorid, Cetyltrimethylammoniumchlorid, Behenyltrimethylammoniumchlorid, Lauryltrimethylammoniumchlorid, Tetradecyltrimethylammoniumchlorid. Lauryloxy-2-hydroxy-propyl-trimethylammoniumchlorid, Cocoyltrimethylammoniumchlorid, Sojatrimethylammoniumchlorid und Stearyltrimethylammoniumchlorid sowie Mischungen dieser Verbindungen besonders bevorzugt sind.

Im Hinblick auf eine besonders hohe Lagerstabilität sind Mittel besonders bevorzugt, bei denen die Alkylkettenlänge der Komponenten Fettalkohol (a), Fettalkoholalkoxylat (c) und quaternäre Ammoniumverbindung (d) jeweils gleich sind (z.B. sowohl der Fettalkohol als auch das Fettalkoholalkoxylat als auch das Ammoniumquat haben als Alkylgruppe eine Cetyl- oder Stearylgruppe, wobei das Ammoniumquat auch zwei Cetyl- oder Stearylgruppen aufweisen kann).

Vorzugsweise ist das erfindungsgemäße Färbemittel frei von üblichen Perlglanzbildnern.

Das erfindungsgemäße Färbemittel enthält vorzugsweise Oxidationsfarbstoffvorstufen, bei denen die Färbung unter Einwirkung von Oxidationsmitteln, wie zum Beispiel Wasserstoffperoxid, oder in Gegenwart von Luftsauerstoff erzeugt wird.

Als geeignete Oxidationsfarbstoffvorstufen können beispielsweise die folgenden Entwicklersubstanzen und Kupplersubstanzen und mit sich selbst kuppelnden Verbindungen genannt werden:
(i) Entwicklersubstanzen: 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 1,4-Diamino-2-(2-hydroxyethoxy)-benzol, 2-(2-(Acetylamino)ethoxy)-1,4-diaminobenzol, 4-Phenylamino-anilin, 4-Dimethylamino-anilin, 4-Diethylaminoanilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methylanilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluorphenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1H-pyrazol, 4,5-Diamino-1-pentyl-1H-pyrazol, 4,5-Diamino-1-(phenylmethyl)-1H-pyrazol, 1,2-Bis(4,5-diamino-1H-pyrazol-1-yl)-ethan, 4,5-Diamino-1-((4-methoxyphenyl)methyl)-1H-pyrazol, 2-Amino-phenol, 2-Amino-6-methyl-phenol, 2-Amino-5-methylphenol, sowie die in der WO 02/46165 A1, DE 101 09 807 A1 und EP 0 740 931 A1 genannten Diaminopyrazolderivate, allein oder im Gemisch miteinander.
(ii) Kupplersubstanzen: N-(3-Dimethylamino-phenyl)-hamstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxybenzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Aminophenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 2-Methyl-1-naphthol, 1,5-Dihydroxy-naphthalin, 1,7-Dihydroxy-naphihalin, 2,3-Dihydroxy-naphthalin, 2,7-Dihydroxy-naphthalin, 2-Methyl-1-naphthol-acetat, 1,3-Dihydroxybenzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxybenzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion, allein oder im Gemisch miteinander.
(iii) Mit sich selbst kuppelnde Verbindungen: 2-Amino-5-methylphenol, 2-Amino-6-methylphenol, 2-Amino-5-ethoxyphenol oder 2-Propylamino-5-aminopyridin.

Selbstverständlich können die Oxidationsfarbstoffvorstufen, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Die Gesamtmenge der in dem erfindungsgemäßen Mittel enthaltenen Oxidationsfarbstoffvorstufen beträgt etwa 0,01 bis 12 Gewichtsprozent, insbesondere etwa 0,2 bis 6 Gewichtsprozent.

Die vorgenannten Oxidationsfärbmittel können zur Erzielung von bestimmten Farbtönen zusätzlich zu den Oxidationsfarbstoffvorstufen natürlich vorkommende und/oder synthetische direktziehende Farbstoffe enthalten.

Als geeignete natürliche oder synthetische direktziehende Farbstoffe können beispielsweise sogenannte Pflanzenfarbstoffe wie Henna oder Indigo,Triphenylmethanfarbstoffe, aromatische Nitrofarbstoffe, Azofarbstofte, Chinonfarbstoffe, Dispersionsfarbstoffe und kationische oder anionische Farbstoffe verwendet werden.

Die Gesamtmenge der direktziehenden Farbstoffe beträgt in dem erfindungsgemässen Mittel etwa 0,01 bis 7 Gewichtsprozent, vorzugsweise etwa 0,2 bis 4 Gewichtsprozent.

Weitere zur Haarfärbung bekannte und übliche Farbstoffe, die in dem erfindungsgemässen Färbemittel enthalten sein können, sind unter anderem in E. Sagarin, "Cosmetics, Science and Technology", Interscience Publishers Inc., New York (1957), Seiten 503 ff. sowie H. Janistyn, "Handbuch der Kosmetika und Riechstoffe", Band 3 (1973), Seiten 388 ff. und K. Schrader "Grundlagen und Rezepturen der Kosmetika", 2. Auflage (1989), Seiten 782-815 beschrieben.

Obwohl Oxidationsfärbemittel bevorzugt sind, ist es selbstverständlich ebenfalls möglich, dass das erfindungsgemässe Färbemittel in Form eines nicht-oxidativen Färbemittels auf Basis der vorstehend genannten direktziehenden Farbstoffen vorliegt

Die Gesamtmenge der direktziehenden Farbstoffe beträgt in diesen nicht-oxidativen Färbemitteln etwa 0,01 bis 10 Gewichtsprozent, vorzugsweise etwa 0,2 bis 5 Gewichtsprozent.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, beispielsweise Ethylendiaminotetraacetat oder Nitriloessigsäure, Netzmittel, Emulgatoren, Verdicker und Pflegestoffe enthalten sein, wobei die Parfümöle in einer Menge von etwa 0,001 bis 1 Gewichtsprozent eingesetzt werden, während die Antioxidantien und Komplexbildner jeweils in einer Menge von von etwa 0,001 bis 0,5 Gewichtsprozent eingesetzt werden.

Selbstverständlich kann das vorstehend beschriebene Färbemittel gegebenenfalls weitere, für Färbemittel übliche Zusätze, wie zum Beispiel Lösungsmittel wie Wasser, niedere aliphatische Alkohole, beispielsweise Ethanol, n-Propanol und Isopropanol oder Glykole wie Glycerin und 1,2-Propandiol, weiterhin (zusätzlich zu den vorgenannten Fettalkoholalkoxylaten und Ammoniumquats) Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, oxethylierte Fettalkohole, oxethylierte Nonylphenole, oxethylierte Fettsäureester, ferner Verdicker wie Stärke oder Cellulosederivate, Parfüme, Haarvorbehandlungsmittel, Konditionierer, Haarquellmittel, Konservierungsstoffe, weiterhin Vaseline, Paraffinöl und Fettsäuren sowie ausserdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von 0,1 bis 30 Gewichtsprozent und die Pflegestoffe in einer Konzentration von 0,1 bis 5,0 Gewichtsprozent.

Der pH-Wert des erfindungsgemäßen Färbemittels liegt bei oxidativen Färbemitteln auf der Basis von Oxidationsfarbstoffvorstufen in einem Bereich von etwa 6 bis 12, vorzugsweise 9 bis 11, wobei der pH-Wert des gebrauchsfertigen Oxidationsfärbemittels (das heißt der Mischung des erfindungsgemäßen Färbemittels mit dem Oxidationsmittel) etwa 5,5 bis 10, vorzugsweise 6 bis 9, beträgt. Bei nicht-oxidativen Färbemitteln auf der Basis von direktziehenden Farbstoffen liegt der pH-Wert im Bereich von etwa 5 bis 10, vorzugsweise 6 bis 9.

Je nach Zusammensetzung und gewünschtem pH-Wert des Färbemittels erfolgt die Einstellung des pH-Wertes vorzugsweise mit Ammoniak oder organischen Aminen, wie zum Beispiel Glucaminen, Aminomethylpropanol, Monoethanolamin oder Triethanolamin, anorganischen Basen, beispielsweise Natriumhydroxid, Kaliumhydroxid, Natriumcarbonat oder Calciumhydroxid, beziehungsweise organischen oder anorganischen Säuren, wie zum Beispiel Milchsäure, Zitronensäure, Essigsäure oder Phosphorsäure.

Das erfindungsgemäße Mittel wird vorzugsweise in Form einer wässrigen oder wässrig-alkoholischen Zubereitung, beispielsweise als verdickte Lösung, als Emulsion, als Creme oder als Gel, konfektioniert.

Für die Anwendung zur oxidativen Färbung vermischt man das vorstehend beschriebene Färbemittel unmittelbar vor dem Gebrauch mit einem Oxidationsmittel und trägt eine für die Färbung ausreichende Menge, in der Regel etwa 60 bis 200 Gramm, der gebrauchsfertigen Zubereitung auf die Faser auf.

Als Oxidationsmittel zur Entwicklung der Färbung kommen hauptsächlich Wasserstoffperoxid oder dessen Additionsverbindungen an Harnstoff, Melamin oder Natriumborat in Form einer 1- bis 12prozentige, vorzugsweise 1,5- bis 6prozentigen wässrigen Lösung in Betracht. Das Mischungsverhältnis von Färbemittel zu Oxidationsmittel ist abhängig von der Konzentration des Oxidationsmittels und beträgt in der Regel etwa 5:1 bis 1:5, bevorzugt 2:1 bis 1:2 und insbesondere 1:1, wobei der Gehalt an Oxidationsmittel in der gebrauchsfertigen Zubereitung vorzugsweise etwa 0,5 bis 8 Gewichtsprozent, insbesondere 1 bis 4 Gewichtsprozent, beträgt. Sofern das erfindungsgemäße Färbemittel keine Oxidationsfarbstoffvorstufen enthält (nicht-oxidatives Färbemittel) beziehungsweise Oxidationsfarbstoffvorstufen enthält, welche mit Luftsauerstoff leicht oxidierbar sind, kann es ohne vorheriges Vermischen mit einem Oxidationsmittel direkt auf die Keratinfaser aufgetragen werden. Es ist jedoch auch möglich, zwecks einer gleichzeitigen Aufhellung der Faser oder einer schnelleren Oxidation der Farbstoffe diese Mittel vor der Anwendung mit einem Oxidationsmittel zu vermischen.

Man läßt das gebrauchsfertige Färbemittel bei etwa 15 bis 50 °C etwa 10 bis 45 Minuten, vorzugsweise etwa 15 bis 30 Minuten lang, auf die Faser (zum Beispiel menschliche Haare) einwirken, spült sodann die Faser mit Wasser aus und trocknet sie. Gegebenenfalls wird im Anschluß an diese Spülung mit einem Shampoo gewaschen und eventuell mit einer schwachen organischen Säure, wie zum Beispiel Weinsäure, nachgespült. Anschließend wird die Faser getrocknet.

Ein mit der erfindungsgemäßen Zusammensetzung hergestelltes Färbemittel erfüllt die in Bezug auf die Hafteigenschaften, das Auftrageverhalten und die Viskositätseinstellung gestellten Anforderungen in hervorragenderer Weise. Zudem ist das mit dem erfindungsgemäßen Färbemittel erzielte Pflegeergebnis nach dem Ausspülen deutlich besser als bei Mitteln gemäß dem Stand der Technik. Weiterhin besitzen die erfindungsgemäßen Färbemittel eine gleichmäßige Konsistenz und eine sehr kosmetische perlmuttglänzende Anmutung ("Perlglanzeffekt").

Die nachfolgenden Beispiele sollen den Gegenstand der Erfindung näher erläutern ohne diesen hierauf zu beschränken.

### Beispiele

### Beispiel 1: Oxidationshaarfärbemittel, cremeförmig

| | |
|---|---|
| 6,0000 g | Stearylalkohol |
| 5,0000 g | Cetylalkohol |
| 8,0000 g | Kokosnussfettsäuremonoethanolamid (Oramide® ML115 der Firma Seppic/FR) |
| 4,0000 g | Steareth-20 (Volpo® S20 der Firma Croda/GB) |
| 5,0000 g | Stearyltrimethylammoniumchlorid (Arquad® 18-50, Firma Akzo Nobel/FR) |
| 1,3620 g | 4-Aminophenol |
| 0,5000 g | 1-Naphthol |
| 0,0136 g | Resorcin |
| 0,0034 g | 2-Amino-6-chlor-4-nitrophenol |
| 12,0000 g | Ammoniak, 25 %ige wässrige Lösung |
| 1,0000 g | Ethylendiaminoteraacetat-Dinatriumsalz |
| 1,0000 g | Ascorbinsäure |
| ad 100,0000 g | Wasser |

50 g des vorstehenden Haarfärbemittels mit Perlmuttglanz werden unmittelbar vor Gebrauch mit 50 g einer 6prozentigen wässrigen Wasserstoffperoxidlösung vermischt. Es wird eine homogene, perlmuttgänzende, kosmetisch anmutende Färbezubereitung erhalten. Das so erhaltene Gemisch wird anschließend auf blonde Naturhaare aufgetragen. Nach einer Einwirkungszeit von 30 Minuten bei 40 °C wird das Haar mit Wasser gespült und getrocknet. Das Haar erhält eine leuchtende, kupferrote Färbung.

### Beispiel 2: Creme-Oxidationshaarfärbemittel zur Hellerfärbung

### Komponente (A): Cremförmige Farbträgermasse

| | |
|---|---|
| 6,00 g | Behenylalkohol |
| 8,00 g | Stearylalkohol |
| 8,00 g | Kokosnussfettsäuremonoethanolamid (Comperlan® 100 der Firma Cognis/DE) |
| 6,00 g | Steareth-10 (Brij® 76 der Firma ICI Surfactants) |
| 1,00 g | Ölsäure |
| 4,00 g | Behenyltrimethylammoniumchlorid (Genamin® KDMP der Firma Clariant/CH) |
| 0,50 g | p-Phenylendiamin |
| 0,07 g | Resorcin |
| 1,00 g | Ethylendiamintetraessigsäure-Dinatriumsalz |
| 8,00 g | Ammoniak, 25 %ige wässrige Lösung |
| 8,00 g | Ethanol |
| ad 100,00 g | Wasser |

### Komponente (B): Wasserstoffperoxid-Emulsion

| | |
|---|---|
| 10,0 g | Cetylstearylalkohol |
| 1,5 g | Cholesterin |
| 4,0 g | Natriumlaurylalkohol-diglykolethersulfat, 28 %ige wässrige Lösung |
| 35,0 g | Wasserstoffperoxid, 35 %ige wässrige Lösung |
| 0,3 g | Parfüm |
| ad 100,0 g | Wasser |

Man vermischt vor dem Gebrauch 40 g der flüssigen, perlmuttglänzenden Farbträgermasse (A) mit 80 g der Wasserstoffperoxid-Emulsion (B), entsprechend einem Mischungsverhältnis von (A):(B) von 1:2, und trägt 120 g des erhaltenen perlmuttglänzenden Gemisches auf graues, menschliches Haar auf. Nach einer Einwirkungszeit von 20 Minuten bei Raumtemperatur wird das Haar mit Wasser ausgespült getrocknet. Das so behandelte Haar ist vom Haaransatz bis zu den Haarspitzen gleichmäßig hellbraun gefärbt. Das erfindungsgemäße Mittel ist leicht auftragbar und läuft nicht vom Haar ab.

### Beispiel 3: Oxidationshaarfärbemittel, cremeförmig

| | |
|---|---|
| 4,00 g | Cetylstearylakohol |
| 5,00 g | Behenylalkohol |
| 12,00 g | Kokosnussfettsäuremonoethanolamid (Rewomid® C212 der Firma Goldschmidt/DE) |
| 2,00 g | Steareth-20 (Alkanol S20P der Firma Goldschmidt) |
| 5,00 g | Distearyldimethylammoniumchlorid (Arquad® 2HAT-75 der Firma Akzo Nobel) |
| 8,00 g | Monoethanolamin |
| 1,30 g | 1-Methyl-2,5-diaminobenzol |
| 1,00 g | Bienenwachs |
| 0,65 g | Resorcin |
| 0,50 g | Keratinhydrolysat |
| 0,50 g | Seidenproteinhydrolysat |
| 0,52 g | 2-Amino-6-chlor-4-nitrophenol |
| 1,00 g | Ethylendiamintetraessigsäure-Dinatriumsalz |
| 0,30 g | Ascorbinsäure |
| ad 100,00 g | Wasser |

50 g des vorstehenden Haarfärbemittels mit Perlmuttglanz werden unmittelbar vor Gebrauch mit 50 g einer 12prozentigen wässrigen Wasserstoffperoxidiösung vermischt. Der Perlmuttglanz bleibt auch nach dem Vermischen erhalten. Das Gemisch wird sodann auf blonde Naturhaare aufgetragen und nach einer Einwirkungszeit von 30 Minuten bei 40 °C mit Wasser wieder aus dem Haar ausgespült. Nach dem Trocknen der Haare wird ein gleichmäßiger, kräftiger Braunton erhalten.

### Beispiel 4: nicht-oxidatives Haarfärbemittel

| | |
|---|---|
| 6,000 g | Stearylalkohol |
| 5,000 g | Behenylalkohol |
| 8,000 g | Kokosnussfettsäuremonoethanolamid (Rewomid® C212 der Firma Goldschmidt/DE) |
| 2,000 g | Steareth-20 (Volpo® S20 der Firma Croda/GB) |
| 2,000 g | Distearyldimethylammoniumchlorid |
| 2,000 g | Isopropylalkohol |
| 0,160 g | 4-[Ethyl-(2-hydroxyethyl)amino]-1-[(2-hydroxyethyl)-amino]-2-nitrobenzol-hydrochlorid (HC Blue No. 12) |
| 0,170 g | 4-[(2,3-Dihydroxypropyl)amino]-3-nitro-1-trifluormethyl-benzol (HC Yellow No. 6) |
| 0,012 g | 1-N-Hydroxyethylamino-4-methyl-2-nitrobenzol |
| 0,025 g | 1-Amino-5-chlor-4-[(2,3-dihydroxypropyl)amino]-2-nitrobenzol (HC Red No. 10) |
| 0,010 g | 5-Chlor-1,4-[di(2,3-dihydroxypropyl)amino]-2-nitrobenzol (HC Red No. 11) |
| ad 100,000 g | Wasser |

Die perlglänzende cremeartige Färbemasse wird auf gewaschene und handtuchtrockene blonde Naturhaare aufgetragen und für etwa 20 bis 25 Minuten einwirken gelassen. Anschließend wird die überschüssige Farbe mit Wasser und einem Shampoo herausgwaschen, das Haar getrocknet und gegebenfalls zur Frisur gelegt. Es wird ein schöner, glänzender mittelblonder Ton erreicht.

### Beispiel 5: nicht-oxidatives Haarfärbemittel

| | |
|---|---|
| 3,00 g | Cetylstearylakohol |
| 3,00 g | Stearylalkohol |
| 5,00 g | Kokosnussfettsäurediethanolamid (Rewomid® DC212S der Firma Goldschmidt/DE) |
| 2,00 g | Oteth-30 |
| 2,50 g 7,00 g | Behenyltrimethylammoniumchlorid Ethanol |
| 0,10 g | 1-N-Hydroxyethylamino-4-methyl-2-nitrobenzol |
| 0,35 g | 1-Amino-5-chlor-4-[(2,3-dihydroxypropyl)amino]-2-nitrobenzol (HC Red No. 10) |
| 0,15 g | 5-Chlor-1,4-[di(2,3-dihydroxypropyl)amino]-2-nitrobenzol (HC Red No. 11) |
| 0,20 g | 2-Amino-6-chloro-4-nitrophenol |
| ad 100,00 g | Wasser |

Die perlglänzende cremeartige Färbemasse wird auf gewaschene und handtuchtrockene blonde Naturhaare aufgetragen und für etwa 20 bis 25 Minuten einwirken gelassen. Anschließend wird die überschüssige Farbe mit Wasser und einem Shampoo herausgwaschen, das Haar getrocknet und gegebenfalls zur Frisur eingelegt. Es wird ein schöner, glänzender modischer Rotton erhalten.

### Beispiel 6: Haarfärbemittel

| | |
|---|---|
| 0,33 g | 2,5-Diamino-toluol-sulfat |
| 0,33 g | 2,5-Diamino-phenylethanol-sulfat |
| 0,22 g | N,N-Bis-(β-hydroxyethyl)-p-phenylendiamin-sulfat |
| 0,33 g | 4-Amino-3-methyl-phenol |
| 0,22 g | 2-Aminomethyl-p-aminophenol * HCl |
| 0,05 g | 4,5-Diamino-1-hydroxyethyl-pyrazol-sulfat |
| 0,05 g | 4,5-Diamino-1-(p-methylbenzyl)-pyrazol-sulfat |
| 0,05 g | 4,5-Diamino-1-(1-methylethyl)-pyrazol-sulfat |
| 0,05 g | 4,5-Diamino-1-methyl-pyrazol-sulfat |
| 0,05 g | 4,5-Diamino-1-(4-methoxybenzyl)-pyrazol-sulfat |
| 0,05 g | 4,5-Diamino-1-benzyl-pyrazol-sulfat |
| 0,05 g | 1,2-Bis(4,5-diamino-1-H-pyrazol-1-yl)ethan-sulfat |
| 0,05 g | 4,5-Diamino-1-(4-chlorbenzyl)-pyrazol-sulfat |
| 0,05 g | 4,5-Diamino-1-pentyl-pyrazol-sulfat |
| 0,11 g | 1-Naphtol |
| 0,11 g | 3,4-Diamino-benzoesäure |
| 0,11 g | 1-(β-Hydroxyethylamino)-3,4-methylendioxybenzol * HCl |
| 0,11 g | 2,4-Diamino-1-(β-hydroxyethoxy)benzol-sulfat |
| 0,11 g | 5-Amino-6-chlor-2-methyl-phenol |
| 0,11 g | 1,3-Bis-2,4-(diaminophenoxy)propan * 2HCl |
| 0,11 g | 3-Aminophenol |
| 0,11 g | 4-Chlor-resorcin |
| 0,11 g | 5-Amino-2-methyl-phenol |
| 0,11 g | 2-Amino-4-(β-hydroxyethylamino)-anisol-sulfat |
| 0,11 g | 2,4-Diamino-1-fluor-5-methyl-benzol-sulfat (1:1) |
| 0,11 g | 3,5-Diamino-2,6-dimethoxy-pyridin * 2HCl |
| 0,11 g | Resorcin |
| 0,11 g | 2-Methyl-resorcin |
| 0,11 g | m-Dimethylaminophenylhamstoff |
| 0,02 g | 2-Amino-5-methyl-phenol |
| 0,02 g | 2-Amino-6-chlor-4-nitrophenol |
| 0,02 g | 4-(β-Hydroxyethylamino)-3-nitrophenol |
| 0,02 g | 4-[-3-Hydroxypropylamino]-3-nitro-phenol |
| 0,02 g | N1-(2-Hydroxyethyl)-2-nitro-p-phenylenediamin (HC Red 3) |
| 0,02 g | 4-Amino-3-nitrophenol |
| 0,02 g | 2-Amino-4,6-dinitrophenol, 65%ige wässrige Lösung |
| 0,02 g | 2-Hydroxyethyl-pikraminsäure |
| 0,02 g | 1-N-Hydroxyethylamino-4-methyl-2-nitrobenzol |
| 0,02 g | 6-Ethylamino-2-chlor-4-nitro-phenol |
| 0,02 g | 2,6-Diamino-3-((pyridin-3-yl)azo)pyridin |
| 0,02 g | 4-Nitrophenyl-aminoethylharnstoff |
| 0,01g | 1-Amino-5-chlor-4-((2,3 dihydroxypropyl)amino)-2-nitrobenzol |
| 4,00 g | Cetylstearylakohol |
| 5,00 g | Behenylalkohol |
| 12,00 g | Kokosnussfettsäuremonoethanolamid |
| 2,00 g | Steareth-20 |
| 5,00 g | Distearyldimethylammoniumchlorid |
| 8,00 g | Monoethanolamin |
| 3,50 g | Ammoniak, 25%ige wässrige Lösung |
| 1,00 g | Bienenwachs |
| 0,50 g | Keratinhydrolysat |
| 0,50 g | Seidenproteinhydrolysat |
| 1,00 g | Ethylendiamintetraessigsäure-Dinatriumsalz |
| 0,30 g | Ascorbinsäure |
| ad 100,00 g | Wasser |

50 g des vorstehenden Haarfärbemittels mit Perlmuttglanz werden unmittelbar vor Gebrauch mit 50 g einer 12prozentigen wässrigen Wasserstoffperoxidlösung vermischt. Der Perlmuttglanz bleibt auch nach dem Vermischen erhalten. Das Gemisch wird sodann auf blonde Naturhaare aufgetragen und nach einer Einwirkungszeit von 30 Minuten bei 40 °C mit Wasser wieder aus dem Haar ausgespült. Nach dem Trocknen der Haare wird ein gleichmäßiger, kräftiger Schwarzbraunton erhalten.

Alle in der vorliegenden Anmeldung genannten Prozentangaben stellen, soweit nicht anders angegeben, Gewichtsprozente dar.

## Patentansprüche

1. Mittel zur Färbung von Keratinfasern auf der Basis von Oxidationsfarbstoffvorstufen und/oder direktziehenden Farbstoffen, **dadurch gekennzeichnet, dass** es eine Kombination aus
a) mindestens einem langkettigem Fettalkohol,
b) mindestens einem Alkanolamid,
c) mindestens einem Fettalkoholalkoxylat, und
d) mindestens einer quaternären Ammoniumverbindung der Formel (II) oder (III), in denen R1 und R2 unabhängig voneinander gleich einer geradkettigen oder verzweigten C8-C24-Alkylgruppe sind, 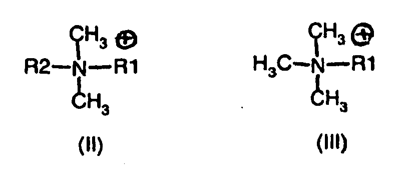 enthält,
wobei das Mengenverhältnis von Fettalkohol (a) zu Alkanolamid (b) 0,5:1,5 bis 1,5:0,5 beträgt.

2. Mittel nach Anspruch 1, **dadurch gekennzeichnet, dass** es eine Kombination aus
a) 2 bis 12 Gewichtsprozent mindestens eines langkettigem Fettalkohols,
b) 2 bis 12 Gewichtsprozent mindestens eines Alkanolamides,
c) 0,5 bis 10 Gewichtsprozent mindestens eines Fettalkoholalkoxylates,
und
d) 0,5 bis 10 Gewichtsprozent mindestens einer quaternären
Ammoniumverbindung
enthält.

3. Mittel nach Anspruch 1 oder 2, **dadurch gekennzeichnet, dass** das Mengenverhältnis von Fettalkohol (a) zu Alkanolamid (b) gleich 0,8:1,2 bis 1,2:0,8 ist.

4. Mittel nach einem der Ansprüche 1 bis 3, **dadurch gekennzeichnet, dass** Mengenverhältnis von Fettalkoholalkoxylat (c) zu quaternärer Ammoniumverbindung (d) gleich 0,5:1,5 bis 1,5:0,5, insbesondere 0,8:1,2 bis 1,2:0,8, ist.

5. Mittel nach einem der Ansprüche 1 bis 4, **dadurch gekennzeichnet, dass** der langkettige Fettalkohol (a) ein geradkettiger oder verzweigter Fettalkohole mit 8 bis 30 Kohlenstoffatomen ist.

6. Mittel nach Anspruch 5, **dadurch gekennzeichnet, dass** der langkettige Fettalkohol (a) ausgewählt ist aus Cetylakohol, Stearylalkohol und Behenylalkohol sowie Mischungen dieser Fettalkohole.

7. Mittel nach einem der Ansprüche 1 bis 4, **dadurch gekennzeichnet, dass** das Alkanolamid (b) ein Monoalkanolamid, Dialkanolamid oder Esteramid, insbesondere ein N-Acylderivat des Monoethanolamins oder Diethanolamins, ist.

8. Mittel nach Anspruch 7, **dadurch gekennzeichnet, dass** das Alkanolamid (a) ausgewählt ist aus Laurinsäuremonoethanolamid, Kokosnussfettsäuremonoethanolamid, Kokosnussfettsäurediethanolamid, Stearinsäuremonoethanolamid, Palmitinsäuremonoethanolamid und Ölsäuremonoethanolamid sowie Mischungen dieser Alkanolamide.

9. Mittel nach einem der Ansprüche 1 bis 4, **dadurch gekennzeichnet, dass** das Fettalkoholalkoxylat (c) ein Fettalkoholpolyglykoether der Formel (I)
R-(OCH₂CH₂)ₓ-OH (I),
mit R gleich einer geradkettigen oder verzweigten C8-C24-Alkylgruppe oder einer geradkettigen oder verzweigten C8-C24-Alkenylgruppe und x gleich 2 bis 300, ist.

10. Mittel nach Anspruch 9, **dadurch gekennzeichnet, dass** das Fettalkoholalkoxylat (c) ausgewählt ist aus Polyethylenglykolethern des Stearylalkohols mit 10 bis 50 Ethylenoxideinheiten im Molekül.

11. Mittel nach einem der Ansprüche 1 bis 4, **dadurch gekennzeichnet, dass** die quaternäre Ammoniumverbindung (d) der Formel (II) oder (III) ausgewählt ist aus Verbindungen, in denen R1 und R2 unabhängig voneinander gleich einer geradkettigen oder verzweigten C14-C22-Alkylgruppe sind.

12. Mittel nach Anspruch 11, **dadurch gekennzeichnet, dass** das die quaternäre Ammoniumverbindung (d) ausgewählt ist aus Distearyldimethylammonium-chlorid, 2-Hexyldecyltrimethylammoniumchlorid, Cetyltrimethyl-ammoniumchlorid, Behenyltrimethylammoniumchlorid, Lauryltrimethyl-ammoniumchlorid, Lauryloxy-2-hydroxypropyltrimethylammoniumchlorid, Cocoyltrimethylammoniumchlorid, Sojatrimethylammoniumchlorid und Stearyltrimethylammoniumchlorid sowie Mischungen dieser Verbindungen.

13. Mittel nach einem der Ansprüche 1 bis 12, **dadurch gekennzeichnet, dass** es ein Oxidationsfärbemittel ist und 0,01 bis 12 Gewichtsprozent Oxidationsfarbstoffvorstufen enthält.

14. Mittel nach Anspruch 13, **dadurch gekennzeichnet, dass** es 0,01 bis 7 Gewichtsprozent direktziehende Farbstoffe enthält.

15. Mittel nach einem der Ansprüche 1 bis 12, **dadurch gekennzeichnet, dass** es ein nicht-oxidatives Färbemittel ist und 0,01 bis 10 Gewichtsprozent direktziehende Farbstoffe enthält.

16. Mittel nach einem der Ansprüche 1 bis 15, **dadurch gekennzeichnet, dass** es ein Haarfärbemittel ist.

17. Verwendung einer Kombination aus
a) mindestens einem langkettigem Fettalkohol,
b) mindestens einem Alkanolamid,
c) mindestens einem Fettalkoholalkoxylat, und
d) mindestens einer quaternären Ammoniumverbindung der Formel (II) oder (III), in denen R1 und R2 unabhängig voneinander gleich einer geradkettigen oder verzweigten C8-C24-Alkylgruppe sind, 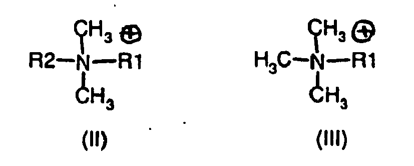 enthält,
wobei das Mengenverhältnis von Fettalkohol (a) zu Alkanolamid (b) 0,5:1,5 bis 1,5:0,5 beträgt,
zur Herstellung von Färbemitteln für Keratinfasern mit Perlmuttglanz auf der Basis von Oxidationsfarbstoffvorstufen und/oder direktziehenden Farbstoffen.

18. Verwendung nach Anspruch 17, **dadurch gekennzeichnet, dass** eine Kombination aus
a) 2 bis 12 Gewichtsprozent mindestens eines langkettigem Fettalkohols,
b) 2 bis 12 Gewichtsprozent mindestens eines Alkanolamides,
c) 0,5 bis 10 Gewichtsprozent mindestens eines Fettalkoholalkoxylates, und
d) 0,5 bis 10 Gewichtsprozent mindestens einer quaternären Ammoniumverbindung,
verwendet wird.

19. Verwendung nach Anspruch 17 oder 18, **dadurch gekennzeichnet, dass** das Mengenverhältnis von Fettalkohol (a) zu Alkanolamid (b) gleich 0,8:1,2 bis 1,2:0,8 ist.

20. Verwendung nach einem der Ansprüche 17 bis 19, **dadurch gekennzeichnet, dass** Mengenverhältnis von Fettalkoholalkoxylat (c) zu quaternärer Ammoniumverbindung (d) gleich 0,5:1,5 bis 1,5:0,5, insbesondere 0,8:1,2 bis 1,2:0,8, ist.

## Claims

1. An agent for coloring keratin fibers based on oxidative colorant precursors and/or direct-penetrating colorants, **characterized in that** it contains a combination of
a) at least one long-chain fatty alcohol
b) at least one alkanol amide
c) at least one fatty alcohol alkoxylate, and
d) at least one quaternary ammonium compound of Formula (II) or (III), in which R1 and R2, independently of one another, are the equivalent of a straight-chain or branched C8-C24 alkyl group, 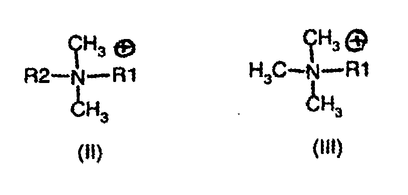 wherein the quantity ratio between the fatty alcohol (a) and the alkanol amide (b) is from 0.5:1.5 to 1.5:0.5.

2. The agent according to Claim 1, **characterized in that** it contains a combination of
a) 2 to 12% by weight of at least one long-chain fatty alcohol
b) 2 to 12% by weight of at least one alkanol amide
c) 0.5 to 10% by weight of at least one fatty alcohol alkoxylate, and
d) 0.5 to 10% by weight of at least one quaternary ammonium compound.

3. The agent according to Claim 1 or 2, **characterized in that** the quantity ratio between the fatty alcohol (a) and the alkanol amide (b) is equal to 0.8:1.2 to 1.2:0.8.

4. The agent according to any of Claims 1 to 3, **characterized in that** the quantity ratio between the fatty alcohol alkoxylate (c) and the quaternary ammonium compound (d) is equal to 0.5:1.5 to 1.5:0.5, or particularly 0.8:1.2 to 1.2:0.8.

5. The agent according to any of Claims 1 to 4, **characterized in that** the long-chain fatty alcohol (a) is a straight-chain or branched fatty alcohol having 8 to 30 carbon atoms.

6. The agent according to Claim 5, **characterized in that** the long-chained alcohol (a) is selected from cetyl alcohol, stearyl alcohol, and behenyl alcohol, as well as mixtures of these fatty alcohols.

7. The agent according to any of Claims 1 to 4, **characterized in that** the alkanol amide (b) is a monoalkanol amide, dialkanol amide, or ester amide, particularly an N-acyl derivative of the monoethanol amine or diethanol amine.

8. The agent according to Claim 7, **characterized in that** the alkanol amide (a) is selected from lauric acid monoethanol amide, coconut fatty acid monoethanol amide, coconut fatty acid diethanol amide, stearic acid monoethanol amide, palmitic acid monoethanol amide, and oleic acid monoethanol amide, as well as mixtures of these alkanol amides.

9. The agent according to any of Claims 1 to 4, **characterized in that** the fatty alcohol alkoxylate (c) is a fatty alcohol polyglycol ether of Formula (I)
R-(OCH₂CH₂)ₓ-OH (I),
where R is the equivalent of a straight-chain or branched C8-C24 alkyl group or a straight-chain or branched C8-C24 alkenyl group, and x equals 2 to 300.

10. The agent according to Claim 9, **characterized in that** the fatty alcohol alkoxylate (c) is selected from polyethylene glycol ethers of the stearyl alcohol having 10 to 50 ethylene oxide units in the molecule.

11. The agent according to any of Claims 1 to 4, **characterized in that** the quaternary ammonium compound (d) of Formula (II) or (III) is selected from compounds in which R1 and R2, independently of one another, are the equivalent of a straight-chain or branched C 14-C22 alkyl group.

12. The agent according to Claim 11, **characterized in that** the quaternary ammonium compound (d) is selected from distearyl dimethyl ammonium chloride, 2-hexyldecyl trimethyl ammonium chloride, cetyl trimethyl ammonium chloride, behenyl trimethyl ammonium chloride, lauryl trimethyl ammonium chloride, lauryloxy-2-hydroxypropyl trimethyl ammonium chloride, cocoyltrimethyl ammonium chloride, soy trimethyl ammonium chloride, and stearyl trimethyl ammonium chloride, as well as mixtures of these compounds.

13. The agent according to any of Claims 1 to 12, **characterized in that** it is an oxidative colorant and contains 0.01 to 12% by weight oxidative colorant precursors.

14. The agent according to Claim 13, **characterized in that** it contains 0.01 to 7% by weight direct-penetrating colorants.

15. The agent according to any of Claims 1 to 12, **characterized in that** it is a non-oxidative colorant and contains 0.01 to 12% by weight direct-penetrating colorants.

16. The agent according to any of Claims 1 to 15, **characterized in that** it is a hair coloring agent.

17. Use of a combination of
a) at least one long-chain fatty alcohol
b) at least one alkanol amide
c) at least one fatty alcohol alkoxylate, and
d) at least one quaternary ammonium compound of Formula (II) or (III), in which R1 and R2, independently of one another, are the equivalent of a straight-chain or branched C8-C24 alkyl group, 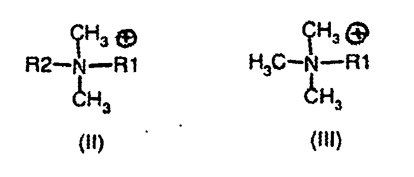 wherein the quantity ratio between the fatty alcohol (a) and the alkanol amide (b) is 0.5:1.5 to 1.5:0.5, for the purposes of producing coloring agents for keratin fibers with pearlescence on the basis of oxidative colorant precursors and/or direct-penetrating colorants.

18. The use according to Claim 17, **characterized in that** a combination of
a) 2 to 12% by weight of at least one long-chain fatty alcohol
b) 2 to 12% by weight of at least one alkanol amide
c) 0.5 to 10% by weight of at least one fatty alcohol alkoxylate, and
d) 0.5 to 10% by weight of at least one quaternary ammonium compound is used.

19. The use according to Claim 17 or 18, **characterized in that** the quantity ratio between the fatty alcohol (a) and the alkanol amide (b) is equal to 0.8:1.2 to 1.2:0.8.

20. The use according to any of Claims 17 to 19, **characterized in that** the quantity ratio between the fatty alcohol alkoxylate (c) and the quaternary ammonium compound (d) is equal to 0.5:1.5 to 1.5:0.5, or particularly 0.8:1.2 to 1.2:0.8.

## Revendications

1. Agent pour la coloration de fibres de kératine basé sur des précurseurs de colorant oxydants et/ou des colorants à pénétration directe, **caractérisé en ce qu'**il contient une combinaison de
a) au moins un alcool gras à longue chaîne
b) au moins un alcanol amide
c) au moins un alcoxylate d'alcool gras, et
d) au moins un composé d'ammonium quaternaire de formule (II) ou (III), dans lequel R1 et R2, indépendamment l'un de l'autre, sont l'équivalent d'un groupe alkyle en C8 à C24 à chaîne linéaire ou ramifié, 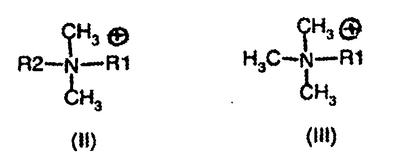 dans lequel le rapport de quantité entre l'alcool gras (a) et l'alcanolamide (b) va de 0,5:1,5 à 1,5:0,5.

2. Agent selon la revendication 1, **caractérisé en ce qu'**il contient une combinaison de
a) 2 à 12 % en poids d'au moins un alcool gras à longue chaîne
b) 2 à 12 % en poids d'au moins un alcanolamide
c) 0,5 à 10 % en poids d'au moins un alcoxylate d'alcool gras, et
d) 0,5 à 10 % en poids d'au moins un composé d'ammonium quaternaire.

3. Agent selon la revendication 1 ou 2, **caractérisé en ce que** le rapport de quantité entre l'alcool gras (a) et l'alcanolamide (b) est égal à 0,8:1,2 à 1,2:0,8.

4. Agent selon l'une quelconque des revendications 1 à 3, **caractérisé en ce que** le rapport de quantité entre l'alcoxylate d'alcool gras (c) et le composé d'ammonium quaternaire (d) est égal à 0,5:1,5 à 1,5:0,5, ou particulièrement 0,8:1,2 à 1,2:0,8.

5. Agent selon l'une quelconque des revendications 1 à 4, **caractérisé en ce que** l'alcool gras à longue chaîne (a) est un alcool gras à chaîne linéaire ou ramifié ayant 8 à 30 atomes de carbone.

6. Agent selon la revendication 5, **caractérisé en ce que** l'alcool à longue chaîne (a) est choisi parmi l'alcool cétylique, l'alcool stéarylique et l'alcool béhénylique, ainsi que des mélanges de ces alcools gras.

7. Agent selon l'une quelconque des revendications 1 à 4, **caractérisé en ce que** l'alcanolamide (b) est un monoalcanolamide, un dialcanolamide, ou un ester amide, particulièrement un dérivé N-acyle de la monoéthanolamine ou de la diéthanolamine.

8. Agent selon la revendication 7, **caractérisé en ce que** falcanolamide (a) est choisi parmi le monoéthanolamide d'acide laurique, le monoéthanolamide d'acide gras de noix de coco, le diéthanolamide d'acide gras de noix de coco, le monoéthanolamide d'acide stéarique, le monoéthanolamide d'acide palmitique et le monoéthanolamide d'acide oléique, ainsi que des mélanges des ces alcanolamides.

9. Agent selon l'une quelconque des revendications 1 à 4, **caractérisé en ce que** l'alcoxylate d'alcool gras (c) est un polyglycol éther d'alcool gras de formule (I)
R-(OCH₂CH₂)ₓ-OH (I),
où R est l'équivalent d'un groupe alkyle en C8-C24 à chaîne linéaire ou ramifié ou d'un groupe alcényle en C8-C24 à chaîne linéaire ou ramifié, et x est égal à 2 à 300.

10. Agent selon la revendication 9, **caractérisé en ce que** l'alcoxylate d'alcool gras (c) est choisi parmi des éthers de polyéthylène glycol de l'alcool stéarylique ayant 10 à 50 motifs oxyde d'éthylène dans la molécule.

11. Agent selon l'une quelconque des revendications 1 à 4, **caractérisé en ce que** le composé d'ammonium quaternaire (d) de formule (II) ou (III) est choisi parmi les composés dans lesquels R1 et R2, indépendamment l'un de l'autre, sont l'équivalent d'un groupe alkyle en C14-C22 à chaîne linéaire ou ramifié.

12. Agent selon la revendication 11, **caractérisé en ce que** le composé d'ammonium quaternaire (d) est choisi parmi le chlorure de distéaryldiméthylammonium, le chlorure de 2-hexyldécyl triméthylammonium, le chlorure de cétyltriméthylammonium, le chlorure de béhényltriméthylammonium, le chlorure de lauryltriméthylammonium, le chlorure de lauryloxy-2-hydroxypropyl triméthylammonium, le chlorure de cocoyltriméthylammonium, le chlorure de soja triméthylammonium et le chlorure de stéaryltriméthylammonium, ainsi que des mélanges de ces composés.

13. Agent selon l'une quelconque des revendications 1 à 12, **caractérisé en ce qu'**il est un colorant oxydant et contient 0,01 à 12 % en poids de précurseurs de colorant oxydants.

14. Agent selon la revendication 13, **caractérisé en ce qu'**il contient 0,01 à 7 % en poids de colorants à pénétration directe.

15. Agent selon l'une quelconque des revendications 1 à 12, **caractérisé en ce qu'**il est un colorant non oxydant et contient 0,01 à 10 % en poids de colorants à pénétration directe.

16. Agent selon l'une quelconque des revendications 1 à 15, **caractérisé en ce qu'**il s'agit d'un agent de coloration capillaire.

17. Utilisation d'une combinaison de
a) au moins un alcool gras à longue chaîne
b) au moins un alcanol amide
c) au moins un alcoxylate d'alcool gras, et
d) au moins un composé d'ammonium quaternaire de formule (II) ou (III), dans lequel R1 et R2, indépendamment l'un de l'autre, sont l'équivalent d'un groupe alkyle en C8 à C24 à chaîne linéaire ou ramifié, 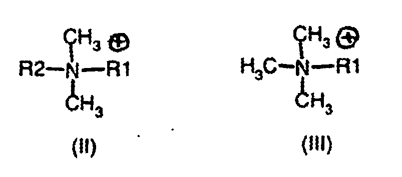 dans laquelle le rapport de quantité entre l'alcool gras (a) et l'alcanolamide (b) va de 0,5:1,5 à 1,5:0,5, dans le but de produire des agents colorants pour fibres de kératine avec une opalescence sur la base des précurseurs de colorant oxydants et/ou des colorants à pénétration directe.

18. Utilisation selon la revendication 17, **caractérisée en ce qu'**une combinaison de
a) 2 à 12 % en poids d'au moins un alcool gras à longue chaîne
b) 2 à 12 % en poids d'au moins un alcanolamide
c) 0,5 à 10 % en poids d'au moins un alcoxylate d'alcool gras, et
d) 0,5 à 10 % en poids d'au moins un composé d'ammonium quaternaire est utilisée.

19. Utilisation selon la revendication 17 ou 18, **caractérisée en ce que** le rapport de quantité entre l'alcool gras (a) et l'alcanolamide (b) est égal à 0,8:1,2 à 1,2:0,8.

20. Utilisation selon l'une quelconque des revendications 17 à 19, **caractérisée en ce que** le rapport de quantité entre l'alcoxylate d'alcool gras (c) et le composé d'ammonium quaternaire (d) est égal à 0,5:1,5 à 1,5:0,5, ou particulièrement 0,8:1,2 à 1,2:0,8.
